# EUROPEAN PATENT APPLICATION

(11) **EP 4 512 883 A1**
(43) Date of publication of application: **26.02.2025**
(21) Application number: 23193573.5
(22) Date of filing: 25.08.2023
(51) Int. Cl.: C12M 1/00, B01F 31/23, C12M 3/06, C12M 1/34

(54) **METHOD OF HARVESTING CELL BROTH, AND MIXING DEVICE**

(71) Applicant: Sartorius Stedim Biotech GmbH, 37079 Göttingen (DE)
(72) Inventor: Heuer, Dennis, 37079 Goettingen (DE); Leupold, Marco, 34302 Guxhagen (DE); Gao, Wei, 34302 Guxhagen (DE); Wolff, Alexander, 37079 Goettingen (DE)
(74) Representative: Prinz & Partner mbB

(57) **Abstract**

A method of harvesting cell broth (12) from a mixing device (10) for mixing the cell broth (12) of a rocking motion bioreactor is shown. The mixing device (10) comprises a rocking motion platform (14), a mixing bag (18) and a processing unit (22). After a harvest process is started, the mixing bag (18) is tilted into a default harvest position and the cell broth (12) is harvested in the default harvest position of the mixing bag (18). If a pause condition is fulfilled, harvesting is paused and the mixing device starts rocking at a defined speed and a defined rocking angle for a predetermined time. After the predetermined time, harvesting in the default harvest position is resumed. Furthermore, a mixing device (10) for mixing cell broth (12) is disclosed.

## Description

The invention relates to a method of harvesting cell broth from a mixing device for mixing cell broth of a rocking motion bioreactor. Further, the invention relates to a mixing device for mixing cell broth of a bioreactor.

Over the last few years, the biopharmaceutical industry has pushed for solutions for more productive and adaptable bioprocessing. Due to rising demand, stiff competition, the emergence of new modalities, and digital transformation, the need for shortened time lines and greater yields of increasingly diverse products has continuously increased. One of the easiest ways to keep up with the heightened demand is to increase productivity in the upstream process. One of the fastest and least disruptive ways to achieve this is to intensify the seed train, which helps overcome time and cost constraints and makes upstream manufacturing more agile. While there are many approaches to seed train intensification, rocking motion bioreactors offer a reliable strategy for performing seed train intensification as part of a perfusion process or, more commonly, a hybrid fed-batch/perfusion process.

Implementing a rocking motion bioreactor is the simplest and most cost-effective opportunity for process intensification. These bioreactors typically consist of a single-use mixing bag placed on a rocking motion platform. The mixing bag contains cell broth which is mixed by gentle rocking movements performed by the rocking motion platform. The rocking movements simultaneously provide aeration required for the cells to grow efficiently and reach high densities.

The gentle wave movement of the rocking motion bioreactor offers a mixing and gassing technology with low shear stress, allowing cultures to reach higher densities without the risk of cell damage by submersing bubbles or foam built up. Process analytical technologies such as sensors and controllers can be combined with the rocking motion system in order to provide a tight control of the cell culture environment.

Furthermore, various working volumes from a few ml to a few 100 I can be processed on the same or at most two or three different rocking motion platforms.

However, in order to harvest the cell broth in the mixing bag, the rocking motion platform needs to be tilted into an inclined or upright harvest position. As a result, the wave induced motion and consequently the mixing stops as soon as the harvest process begins. Since the cell broth is no longer being mixed, there is a chance of the cells in the cell broth sinking to the bottom of the mixing bag forming a sediment such that the cell density differs from region to region. Furthermore, there is a risk that the cells no longer receive the required amount of oxygen such that the cells might die, leading to a batch loss.

Therefore, it is an objective of the invention to provide an improved harvest process, which reduces the risk of a batch loss and generally improves the quality of the harvested cell broth.

This objective is solved by a method of harvesting cell broths from a mixing device for mixing the cell broth of a rocking motion bioreactor. The mixing device comprises a rocking motion platform, a mixing bag and a processing unit. After a harvest process is started, the mixing bag is tilted into a default harvest position and the cell broth is harvested in the default harvest position of the mixing bag. If a pause condition is fulfilled, harvesting is paused and the mixing device starts rocking at a defined speed and a defined rocking angle for a predetermined time. After the predetermined time, harvesting in the default harvest position is resumed.

By remixing the cell broth, the risk of cells dying or a total batch loss is reduced, since all cells in the cell broth are supplied by oxygen again and any sediment that might have started to form is remixed with the media. Similar to the start condition (first trigger condition), which will be explained later, the pause condition (second trigger condition) may only need one sensor signal to reach a critical value. Alternatively, it may be based on multiple sensor values.

The pause condition may for example be based on the dissolved oxygen measured by the sensor such that if the dissolved oxygen is reduced by a certain percentage compared to the start of the harvest process, e.g. 60% saturation down to 10% saturation, harvesting is interrupted and the mixing device starts rocking again. Once the dissolved oxygen has increased again due to the mixing, e.g. from 10% saturation up to 50% saturation, harvesting in the harvest position of the mixing bag is resumed. It is also possible, that an operator is informed that the mixing will start again shortly such that the operator has the chance to decline that process step.

Similarly, the mixing device might start rocking again based on the turbidity or the viable biomass, which can be measured with the BioPAT^{®} ViaMass system by Sartorius Stedim Biotech GmbH, Germany, for example. Hence, if the turbidity increases by a certain amount, e.g. 20 million cells per milliliter up to 40 million cells per milliliter, the rocking motion is started until the turbidity has decreased again, e.g. from 40 million cells per milliliter down to 25 million cells per milliliter.

Preferably, the harvest process is automatically started when a start condition is detected by at least one sensor. This means, an operator is not required to be present in order to start the harvest process.

The start condition may refer to a single signal detected by the at least one sensor that reaches a critical value. Alternatively, the start condition can be based on multiple signals reaching their critical values. In particular, the harvest process may be triggered by any of cell density, turbidity, pH, dissolved oxygen and/or time. The critical value of the cell density may be anywhere between a few million cells per milliliter up to over 100 million cells per milliliter. Similarly, the time until the harvest process starts may range between hours and days, wherein the critical time can be determined up to an accuracy of seconds.

According to a preferred embodiment, the cell broth is harvested while the mixing device continues its rocking motion at the start of the harvest while the cell broth weight is still greater than a first predetermined threshold. When the cell broth weight falls below the first predetermined threshold, the harvest bag is tilted into the default harvest position and harvesting according to the method set out above is started.

This is based on the idea that at the beginning of the harvest process, the cell broth can be harvested while the mixing device continues its rocking motion. Only once the first predetermined threshold is reached, does the mixing bag need to be tilted into the default harvest position in order to continue harvesting the cell broth. Therefore, the basic idea is to continue the rocking motion as long as possible during the harvest process. That way, the formation of a sediment at the bottom of the mixing bag can be prevented and through the rocking motion, the oxygen supply to the cells is ensured. Consequently, the risk of cells dying or even a complete batch loss is decreased. Additionally, the harvest process is automated, such that the different steps are induced based on trigger conditions and/or thresholds without an operator having to start any of the steps, which might induce errors.

The first predetermined threshold corresponds to a minimal working bag weight, meaning that once the cell broth weight falls below the first predetermined threshold, there is not enough cell broth left in the mixing bag to provide the required working conditions.

According to a further embodiment, when the cell broth weight falls below a second predetermined threshold, which is lower than the first predetermined threshold, the harvest is stopped. This further automates the harvest process such that the presence of an operator is not necessarily required in order to proceed to the next step in the harvest process.

The second threshold corresponds to the bag residual weight, which is the weight of the mixing bag itself plus a certain residual of the cell broth.

For example, the first predetermined threshold may correspond to about 20% of the cell broth weight and the second predetermined threshold may correspond to about 8% of the cell broth weight.

According to a preferred embodiment, a value of cell broth weight that is to be harvested, or the amount of biomass that is to be transferred, is entered by an operator via a user interface of the processing unit before the harvest is started. Therefore, the overall cell broth weight may differ from the cell broth weight to be harvested, i. e. not the entire cell broth is harvested. In other words, it is possible to perform a partial harvest.

In order to directly use the harvested cell broth in downstream processes, the harvested cell broth is collected in one or more vessels. However, one or more of those vessels may be used for inoculation to further intensify cell growth in upstream processing. Generally, a variety of vessels may be used, like a harvest vessel, a bottle, a bag, a bioreactor, a (pellet) tank, etc. The vessel chosen to collect the harvested cell broth depends on the intended further use of the cell broth.

According to a preferred embodiment, a signal of the at least one sensor includes any of cell density, capacitance, turbidity, pH, dissolved oxygen, and time. One or more of the signals may be part of the start condition starting the harvest process. Furthermore, they help closely monitoring the harvest process in order to ensure an optimized cell culture environment.

Further, the method may comprise an additional step before the cell broth is harvested, wherein in said additional step, the size of the mixing bag and the corresponding first and second thresholds are determined. The predetermined thresholds are stored in a database accessible by the processing unit. In particular, when a full harvest is intended, this significantly simplifies the process for an operator since all the operator has to do is select the right bag size and the corresponding thresholds are selected automatically by the processing unit.

According to a preferred embodiment, the processing unit is connected to a load cell, such that the mixing bag weight is monitored, preferably continuously monitored, and compared to the first and second predetermined thresholds. Due to the automated and continuous monitoring, it is ensured that the steps are executed at the right moment eliminating the chances of operator-induced failures.

Preferably, when the cell broth is harvested while the mixing device continues its rocking motion, controllers relating to a pump are stopped and the state of each controller for dissolved oxygen, pH, temperature, and rocking motion, if active, is kept unchanged. The pump whose controllers are stopped is not the harvest pump. Instead, it is a pump used to feed nutrients into the cell broth and/or control the pH. Furthermore, the pump may be related to the supply of oxygen and/or other gases or fluids. The controllers obtain their input signals from the at least one sensor and since the sensors are kept active, the pH, temperature, etc. are still continuously monitored.

According to a preferred method, a harvest pump is activated to remove cell broth from the mixing bag before the cell broth is harvested. That is to say, the harvest pump is activated as soon as the harvest process is started when the start condition is fulfilled. By including a harvest pump, the harvest process is sped up - compared to gravity based draining - and becomes more effective as well as more controlled since the cell broth leaves the mixing bag at a defined flow rate and a defined pressure determined by the harvest pump.

According to a further embodiment, when the cell broth weight falls below the first predetermined threshold, the controllers for pH, temperature, and rocking motion are stopped. Since the rocking motion and, hence, the mixing stops once the cell broth weight falls below the first predetermined threshold, there is no need to further monitor these values. pH, temperature, and rocking motion are mainly monitored before and at the beginning of the harvest process in order to ensure mixing at optimized conditions.

According to a further variation, the mixing device comprises gassing actuators, such that when the cell broth weight falls below the first predetermined threshold, the controller for dissolved oxygen is suspended and, preferably, the state of each gassing actuator is kept unchanged. This means, the fed-batch process is effectively stopped and no longer needs to be monitored. In order to simplify the process and minimize the risk of a batch loss, the current supply of the gassing actuators is continued.

In a further variation, when the cell broth weight falls below the second predetermined threshold, the harvest process is stopped and the operator is notified to stop the controller for the dissolved oxygen and any further gassing actuators before dismantling the mixing bag. Alternatively, the operator is asked to confirm the automatic stopping of the controller for dissolved oxygen and any gassing actuators. Since the harvest process has now officially ended, there is no further need for monitoring the dissolved oxygen content and supplying gas and/or nutrients to the remaining cell broth. By notifying the operator, resources can be saved and the time until the next process start is reduced.

According to a preferred variation, an algorithm containing information regarding the individual steps of the method is stored in a memory accessible by the processing unit. This algorithm can be adjusted and stored in the memory by the operator. The information contained by the algorithm includes things like the operation mode, the transfer mode, the harvest pump speed, the rocking interval time, the rocking angle, the target threshold, etc. This means, the algorithm effectively corresponds to a recipe including the harvest steps, the order of the steps, and the time each step takes while being fully customizable by the operator.

According to a further variation, a valve is arranged between the mixing bag and the vessel. The processing unit automatically opens the valve such that the cell broth can be transferred into the vessel when the start condition is fulfilled. Further, the processing unit automatically closes the valve to seal the cell broth in the mixing bag while the pause condition is fulfilled. Therefore, accidental spillage of cell broth into the vessel during the mixing stage of the process is avoided.

If the harvest pump is a peristaltic pump, the valve may be integrated into the peristaltic pump. Such a peristaltic harvest pump includes at least one pair of pinch components. As soon as the liquid transfer via the peristaltic harvest pump is stopped, the pinch components pinch the tubing between the mixing bag and the harvest vessel such that the tubing is effectively sealed.

Furthermore, the processing unit may carry out the method fully automatically or at least semi-automatically with minimal operator interaction after the mixing process is initiated. However, the steps of the harvest process can still be manually activated. By having the start of the harvest process carried out by the processing unit, the operator does not need to be in the laboratory to start the harvest process. This simplifies the operation and improves the timing of the overall process.

According to a preferred variation, when the cell broth weight falls below the second predetermined threshold, cell broth remaining in a tube connecting the mixing bag and the vessel is pumped backwards into the mixing bag. By emptying the tube at the end of the harvest process, it can be ensured that cells will not die in the tube, which might negatively affect the overall process, in particular the following downstream processes.

Furthermore, the objective of the invention is solved by a mixing device for mixing cell broth of a rocking motion bioreactor. The mixing device comprises a rocking motion platform, a harvest pump, a mixing bag containing the cell broth and at least one sensor. The mixing device further comprises a processing unit, preferably a control tower, wherein the processing unit is configured to carry out the method of harvesting cell broth described above. Therefore, the mixing device allows for a fully automated, or at least semi-automated, and improved harvest process.

Further features and advantages of the invention will become apparent from the following description and from the accompanying drawings to which reference is made. In the schematic drawings,
- Figure 1 shows a mixing device, particularly a rocking motion bioreactor in two different positions;
- Figure 2 shows a mixing device connected to a harvest vessel;
- Figure 3 shows a mixing device connected to an inoculation vessel; and
- Figure 4 shows a flow chart of the harvest process.

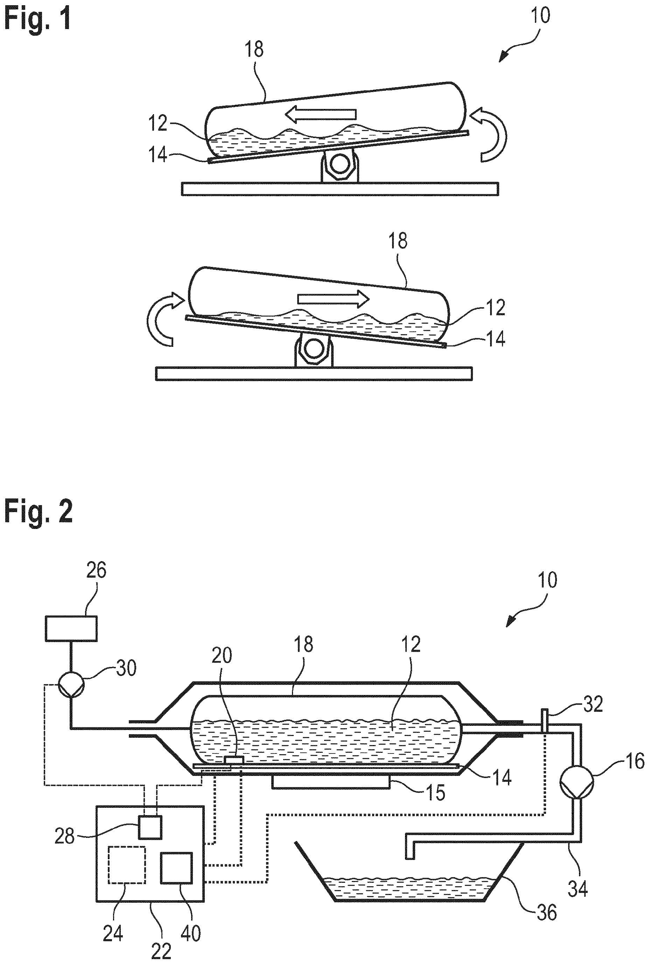

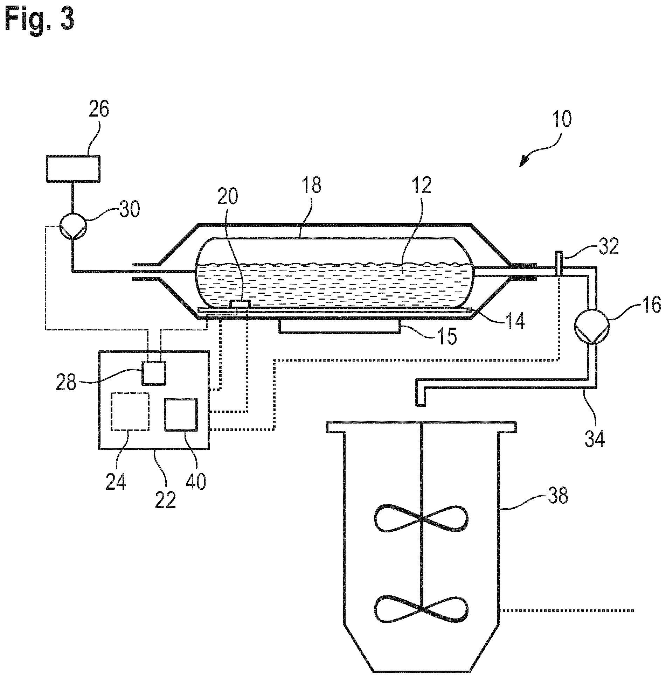

Figure 1 shows a schematic drawing of a mixing device 10, in particular a rocking motion bioreactor, for mixing cell broth 12. The mixing device 10 comprises a rocking motion platform 14, an actuator 15, a harvest pump 16 (shown in Figures 2 and 3) and a mixing bag 18.

Most commonly, the mixing device 10 is part of a process chain assembly and arranged upstream of a larger stirred tank bioreactor 38, as shown in Figure 3.

While the mixing device 10 is most commonly used for seed production for large scale bioreactors 38, it can also be used to supply protein and cell supplies for preclinical purposes as well as GMP production. The seed production for large scale bioreactors 38 may also be for GMP production.

The mixing device 10 is able to work with a variety of different types of cells, including mammalian, insect and plant cells, suspension cells and adherent cells on microcarriers, low to medium density microbial cultures, and shear sensitive cells such as stem cells.

The mixing bag 18 is positioned on the rocking motion platform 14 and can be tilted in either direction, as shown in Figure 1.

In particular, the mixing bag 18 is located on a load cell attached to the rocking motion platform 14 and/or integrated into the rocking motion platform 14. The load cell is configured to continuously measure the weight of the cell broth 12. Alternatively, the load cell may measure the weight of the cell broth 12 at predetermined intervals.

Furthermore, the mixing bag 18 is equipped with at least one sensor 20 and contains the cell broth 12.

While only one sensor 20 is shown in Figure 2, the mixing device 10 may also have multiple sensors 20. Preferably, the sensor(s) 20 include one or more sensors 20 producing signals representative for cell density, turbidity, pH and dissolved oxygen. Moreover, a clock or a timer can also be considered as such a sensor 20, although it does not have to be in contact or close to the cell broth 12.

Generally, the mixing bag 18 used in the mixing device 10 is available in multiple sizes with working volumes ranging from 100 ml to 100 I. The rocking motion platform 14 needs to be suitable for the size of the mixing bag 18 that is used.

The mixing bag 18 is a single-use product such that the mixing device 10 provides consistency in the bioprocess while promoting flexibility and faster processing.

The mixing device 10 further comprises a processing unit 22 (see Figures 2 and 3), which is connected to the rocking motion platform 14 as well as the sensor 20. The processing unit 22 also comprises a memory 24.

Alternatively, the memory 24 may be remote or cloud-based and accessible by the processing unit 22.

Additionally, the processing unit 22 is connected to the load cell, such that the processing unit 22 can monitor the weight of the cell broth 12.

In an alternative embodiment, which is not shown, the processing unit 22 may be a control tower used to execute multiple processes for different bioreactors.

In the embodiments shown in Figures 2 and 3, the processing unit 22 is configured to at least execute the harvest process of the mixing device 10.

The mixing bag 18 is connected to at least one gassing actuator 26, preferably multiple gassing actuators 26, in order to supply oxygen and further gases such as air, nitrogen and carbon dioxide, as required into the mixing bag 18. In order to regulate the cell environment, the gassing actuators are controlled by one or more controllers 28, which may be integrated into the processing unit 22. The controllers 28 receive a signal from the at least one sensor 20, either through a direct connection or through the processing unit 22. Based on the received signal the one or more controllers 28 can adjust one or more actuators, in Figures 2 and 3 a pump 30, such that the cell environment is adjusted.

The mixing device may comprise a controller 28 corresponding to each sensor 20, in particular a pH-controller, a temperature-controller and a DO-controller.

Additionally, the mixing bag 18 may be connected to one or more nutrient supplies, in particular when the mixing device 10 is part of a fed-batch process. Note that actuators responsible for the nutrient supply are not shown in the Figures.

The mixing device 10 and consequently the mixing bag 18 is sealed by a valve 32 such that the cell broth 12 cannot enter the tube 34 connected to a harvest vessel 36 when the valve 32 is closed. The tube 34 is also referred to as harvest line.

The valve 32 is connected to the processing unit 22, such that the processing unit 22 can automatically open and close the valve 32.

Figure 3 shows an alternative embodiment of the mixing device 10, wherein the mixing device 10 is connected to a subsequent bioreactor 38 in a process chain instead of a harvest vessel 36.

In the embodiments shown in Figures 2 and 3, a harvest pump 16 is connected to the tube 34. Besides the harvest vessel 36 shown in Figure 2 and the bioreactor 38 shown in Figure 3, the mixing device 10 may also be connected to a bottle, a bag, a (pellet) tank, etc. Particularly, the bioreactor 38 may be used for inoculation purposes.

The mixing device 10 also includes a user interface 40 in order to adjust and customize a standardized algorithm of the harvest process stored in the memory 24.

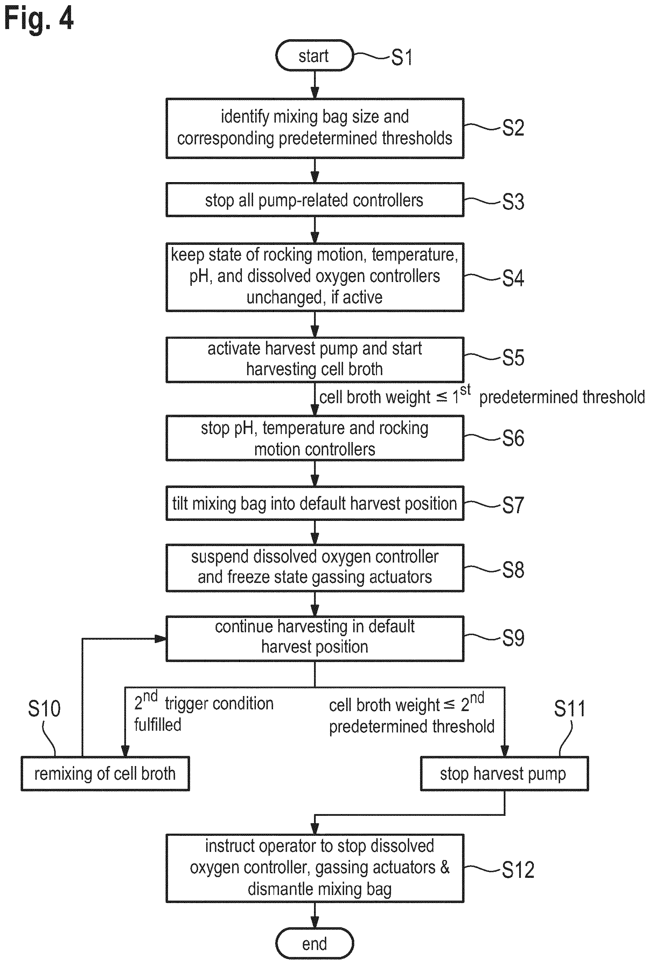

As shown in Figure 4, the algorithm for the harvest process starts or, more accurately, is activated once a first trigger condition (start condition) is fulfilled in a first step S1. This trigger condition may be based on one or more sensor signals that reach a critical value/critical values. Alternatively, the harvest process may be started after a predetermined period of time.

In the following, the harvest process is described in connection with a harvest vessel 36 by way of example since the harvest process itself does not depend on the type of vessel or bioreactor the harvested cell broth is transferred to.

Before the harvest process is started, the mixing device 10 has been mixing the cell broth 12 contained in the mixing bag 18 by performing rocking or wave induced motions. This gentle movement offers a mixing and gassing technology with low shear stress for the cells, which allows cultures to reach higher densities without the risk of cell damage by submersed bubbles of foam built up.

During the mixing process, the cell culture environment is monitored and controlled with multiple sensors 20 and controllers 28. In particular, the mixing device 10 is suitable for fed-batch processes, meaning nutrients and gases, especially oxygen, are provided/introduced to the cell broth 12 during the mixing state.

After the harvest process is started, in a second step S2, the size of the mixing bag 18 is determined. This may be done via the weight determined by the load cell or through an operator input via the user interface 40. Based on the determined mixing bag size, the processing unit 22 determines the corresponding first and second threshold values by selecting the right mixing bag 18 in a database stored in the memory 24.

Additionally, when the first trigger condition is fulfilled, the processing unit 22 opens the valve 32 arranged between the mixing bag 18 and the vessel 36. Before, the valve 32 has been closed in order to prevent cell broth 12 spilling over into the vessel 36. However, in order to start the harvest process, the valve 32 needs to be opened such that cell broth 12 can be transferred into the vessel 36.

In a third step S3, controllers 28 relating to the pump 30 are stopped and the harvest pump 16 is started. In contrast to that, the state of each controller 28 for dissolved oxygen, pH, temperature, and rocking motion, if active, is kept unchanged according to a fourth step S4.

Since the mixing device 10 is still performing its rocking motion, the cell environment needs to be monitored. That way it can be ensured that no sediment is formed at the bottom of the mixing bag 18 and/or cells in the cell broth 12 die.

In a fifth step S5, the harvest pump 16 is activated such that cell broth 12 from the mixing bag 18 is actively transferred into the harvest vessel 36.

Cell broth 12 continues to be harvested while the mixing device 10 executes its rocking motion until the weight of the cell broth 12 falls below the first predetermined threshold. This first predetermined threshold corresponds to a minimal working bag weight, meaning that the amount of cell broth 12 in the mixing bag 18 is below the required amount for the mixing device 10 to perform at an acceptable level.

Consequently, once the cell broth weight falls below the first predetermined threshold, the rocking motion control and consequently the rocking motion of the mixing device 10 is stopped in a sixth step S6. Additionally, the pH and temperature control are stopped as well.

In the next step S7, the mixing bag 18 is tilted into a default harvest position and the cell broth 12 continues to be harvested in said harvest position as indicated in step S9.

Furthermore, in a previous eighth step S8, the controller 28 for dissolved oxygen is suspended and the current state of the gassing actuators 26 is kept unchanged. Since the mixing device 10 is no longer active, there is no need for a continued oxygen end or nutrient supply. However, the sensors 20 are kept active in order to monitor the cell environment.

This is especially important since once a second trigger condition (pause condition) is fulfilled, harvesting is paused and the mixing device 10 restarts its rocking motion at a defined speed and a defined rocking angle as shown in step S10 in Figure 4. The second trigger condition may be based on one or more signals received by the sensor 20, similarly to the first trigger condition.

By restarting the rocking motion of the mixing device 10, the cell broth 12 is mixed again such that a possible sediment of cells at the bottom of the mixing bag 18 is distributed evenly with the media again. Furthermore, the cells are supplied with oxygen again, meaning the dissolved oxygen content should increase, such that a loss of the batch due to cells dying can be prevented.

The mixing device 10 may continue its rocking motion for a predetermined time before resuming harvesting the cell broth 12 according to step S9. Alternatively, the mixing device 10 continues its rocking motion until the trigger conditions are no longer fulfilled, i.e. until the measured signals exceed their critical values again.

Since the mixing device 10 starts its rocking motion and effectively mixing the cell broth 12 contained within the mixing bag 18, the processing unit 22 closes the valve 32 to seal the cell broth 12 in the mixing bag 18 once the second trigger condition is fulfilled. The processing unit 22 also reopens the valve 32 when the harvest according to step S9 is continued.

In case the dissolved oxygen value is used as the second trigger condition, the mixing device 10 would start rocking again when the dissolved oxygen content is reduced by a certain amount compared to the start of the harvest process. For example, the dissolved oxygen content at the start of the harvest process might have been 60% saturation whereas the dissolved oxygen content fulfilling the second trigger condition corresponds to 10% saturation. That means the dissolved oxygen content has significantly decreased during the harvest process. Once the dissolved oxygen content has been regenerated by a certain amount, the mixing device 10 stops its rocking motion, and harvesting according to step S9 is continued. This could mean, that the dissolved oxygen content needs to reach 50% saturation again, for example, before the rocking motion is stopped and the harvesting is continued.

Similarly, turbidity could be used as a trigger condition. Whereas the dissolved oxygen content falls below a critical value, the turbidity would exceed a critical value. For example, the turbidity might increase from 20 million cells per milliliter to 40 million cells per milliliter, leading to the mixing device 10 picking up its rocking motion again. Here, harvesting starts again after the turbidity has decreased to 25 million cells per milliliter again, for example.

It is also possible, that an alert for the operator is generated once the second trigger condition is fulfilled, wherein the alert informs the operator that the mixing device 10 is going to start its rocking motion within a predefined time period, e.g. 20 seconds. During that time, the operator can decline and effectively stop the mixing device 10 from restarting its rocking motion.

The cell broth 12 is harvested according to step S9 until the cell broth weight falls below the second predetermined threshold. This second predetermined threshold corresponds to the bag residual weight and may amount to about 8% of the cell broth volume, for example.

Once the cell broth weight falls below the second predetermined threshold, the harvest pump 16 is stopped as indicated in the next step S11 in Figure 4. The cell broth 12 remaining in the mixing bag 18 is no longer usable since the quality cannot be guaranteed.

In the next step S12, an alert is generated instructing the operator to stop the controller 28 for the dissolved oxygen and any gassing actuators 26 that are still active. It also informs the operator that it is time to dismantle the mixing bag 18 and end the harvest process.

Preferably, the gassing actuators 26 are not stopped automatically and instead need to be manually stopped by an operator before manually dismantling the setup. The continued gassing until the very end of the harvest process can prevent the mixing bag 18 from collapsing and wetting any gas inlet and outlet filters, which are not shown in the Figures. Therefore, any possible disturbances of a possible integrity check of the mixing bag 18 after cultivation is avoided.

It is possible to pump the cell broth 12 remaining in the tube 34 back into the mixing bag 18 by having the harvest pump 16 run backwards for a predefined time or number of revolutions. Afterwards, the harvest pump 16 is stopped in step S11. That way, it is ensured, that the remaining cell broth 12 in the tube 34 does not contain any dead cells which might negatively impact the overall seed process.

Besides the (full) harvest process described above, a partial harvest is also possible. In order to execute a partial harvest, the operator is required to enter a value of cell broth weight that is to be harvested via the user interface 40 before the harvest is started. The harvest process itself does not differ from the harvest process shown in Figure 4. The only difference is in the first and second predetermined threshold, which are adjusted accordingly by the processing unit 22.

Besides the amount to be harvested, all other steps contained in the algorithm can also be adjusted and customized by the operator. In particular, further steps can be added, the order of steps can be changed, and the time each step is allowed to take can be adjusted. Furthermore, each step may be manually activated by the operator via the user interface 40.

Otherwise, the entire harvest process is carried out automatically by the processing unit 22. In particular, when the processing unit 22 is provided by a control tower, the operator is only required to start the mixing process and prepare the tube 34 to establish the connection between the mixing bag 18 and harvest vessel 36. Every other step afterwards can be carried out automatically by the processing unit 22. Consequently, this also means that the operator is not required to be present when the harvest process starts.

### List of Reference signs

- 10: mixing device
- 12: cell broth
- 14: rocking motion platform
- 15: actuator
- 16: harvest pump
- 18: mixing bag
- 20: sensor
- 22: processing unit
- 24: memory
- 26: gassing actuator
- 28: controller
- 30: pump
- 32: valve
- 34: tube
- 36: vessel
- 38: bioreactor
- 40: user interface

## Claims

1. A method of harvesting cell broth (12) from a mixing device (10) for mixing the cell broth (12) of a rocking motion bioreactor, wherein the mixing device (10) comprises a rocking motion platform (14), a mixing bag (18) and a processing unit (22), wherein after a harvest process is started, the mixing bag (18) is tilted into a default harvest position and the cell broth (12) is harvested in the default harvest position of the mixing bag (18), and wherein if a pause condition is fulfilled, harvesting is paused and the mixing device (10) starts rocking at a defined speed and a defined rocking angle for a predetermined time, such that the cell broth (12) is mixed again, and wherein harvesting in the default harvest position is resumed afterwards.

2. The method according to claim 1, wherein the harvest process is automatically started when a start condition is detected by at least one sensor (20).

3. The method according to claim 1 or 2, wherein at the start of the harvest process before the mixing bag (18) is tilted into the default harvest position the cell broth (12) is harvested while the mixing device (10) continues its rocking motion while the cell broth weight is greater than a first predetermined threshold, and wherein the mixing bag (18) is tilted into the default harvest position when the cell broth weight falls below the first predetermined threshold.

4. The method according to claim 3, wherein when the cell broth weight falls below a second predetermined threshold, which is lower than the first predetermined threshold, the harvest is stopped.

5. The method according any of the preceding claims, wherein a value of cell broth weight that is to be harvested, or the amount of biomass that is to be transferred, is entered by an operator via a user interface (40) of the processing unit (22) before the harvest is started.

6. The method according to any of the preceding claims, wherein the harvested cell broth (12) is collected in one or more vessels (36), and wherein preferably one or more of the vessels (36) are used for inoculation.

7. The method according to any of the preceding claims, wherein a signal of the at least one sensor (20) includes any of cell density, capacitance, turbidity, pH, dissolved oxygen, and time.

8. The method according to any of the preceding claims, wherein in a step before step a) the size of the mixing bag (18) and the corresponding first and second thresholds are determined, wherein the predetermined thresholds are stored in a database accessible by the processing unit (22).

9. The method according to any of the preceding claims, wherein during step a) controllers (28) relating to a pump (30) are stopped and the state of each controller (28) for dissolved oxygen, pH, temperature, and rocking motion, if active, is kept unchanged.

10. The method according to any of the preceding claims, wherein when the cell broth weight falls below the first predetermined threshold, the controllers (28) for pH, temperature, and rocking motion are stopped.

11. The method according to any of the preceding claims, wherein the mixing device (10) comprises gassing actuators (26), such that when the cell broth weight falls below the first predetermined threshold, the controller (28) for dissolved oxygen is suspended and, preferably, the state of each gassing actuator (26) is kept unchanged.

12. The method according to any of the preceding claims, wherein when the cell broth weight falls below the second predetermined threshold, the harvest process is stopped and the operator is notified to manually stop or is asked to confirm automatic stopping of the controller (28) for dissolved oxygen and any gassing actuators (26) and dismantle the mixing bag (18).

13. The method according to any of the preceding claims, wherein an algorithm containing information regarding the steps of the method is stored in a memory (24) accessible by the processing unit (22), and wherein the algorithm can be adjusted and stored on the memory (24) by the operator.

14. The method according to any of the preceding claims, wherein a valve (32) is arranged between the mixing bag (18) and the vessel (36), wherein the processing unit (22) opens the valve (32) such that cell broth (12) can be transferred into the vessel (36) when the start condition is fulfilled, and wherein the processing unit (22) closes the valve (32) to seal the cell broth (12) in the mixing bag (18) while the pause condition is fulfilled.

15. A mixing device (10) for mixing cell broth (12) of a rocking motion bioreactor, comprising a rocking motion platform (14), a harvest pump (16), a mixing bag (18) containing the cell broth (12) and at least one sensor (20), wherein the mixing device (10) further comprises a processing unit (22), preferably a control tower, wherein the processing unit (22) is configured to carry out the method of harvesting cell broth (12) according to any of the preceding claims.
